# EUROPEAN PATENT APPLICATION

(11) **EP 2 722 002 A1**
(43) Date of publication of application: **23.04.2014**
(21) Application number: 13188831.5
(22) Date of filing: 16.10.2013
(51) Int. Cl.: A61B 5/055, A61B 8/00

(54) **Method and apparatus for capturing medical images**

(30) Priority: 16.10.2012 KR 20120115035; 10.04.2013 KR 20130039514
(71) Applicant: Samsung Electronics Co., Ltd., Suwon-si, Gyeonggi-do, 443-742 (KR)
(72) Inventor: Song, Myung-sung, Gyeonggi-do (KR); Park, Hae-gweon, Gyeonggi-do (KR); Kim, Se-tae, Seoul (KR)
(74) Representative: Grootscholten, Johannes A.M.

(57) **Abstract**

A method for capturing medical images of an object by using a medical image capturing apparatus for capturing medical images is provided. The method includes: checking a remaining battery capacity of at least one peripheral device that is connected to the medical image capturing apparatus and that receives power from a battery; and providing information relating to a capturing scheme, which is performable by using the medical image capturing apparatus, from among a plurality of capturing schemes which are preset in the medical image capturing apparatus, which information is based on the checked remaining battery capacity.

## Description

### BACKGROUND

### 1. Field

Exemplary embodiments relate to a method and apparatus for capturing medical images, and more particularly, to a medical image capturing apparatus to which at least one peripheral device is connected and a medical image capturing method using the same.

### 2. Description of the Related Art

In the human body, blood vessels, a total length of which is about one hundred thousand kilometers (km), various organs, bones, and other nerves are physiologically connected to each other. Thus, when a human body is abnormal, it is very important to correctly determine and treat the abnormality. However, because an abnormality inside the human body may be difficult to notice, diagnosing a disease before the disease becomes serious is nontrivial. Accordingly, medical image capturing devices, such as X-ray, Computed Tomography (CT), Magnetic Resonance Imaging (MRI), and Positron Emission Tomography (PET) devices, are used to discover, in advance, not only an already-progressing disease but also a risk factor which may cause disease in the future. These medical image capturing devices include various types of equipment which may be employed for observing an internal structure of an organism in order to view structural details, internal tissue, and a fluid flow inside the human body.

Recently, along with the development of wireless communication technology, many wireless peripheral devices have been suggested. Because the wireless peripheral devices do not require wired lines for receiving power and transmitting and receiving data, the wireless peripheral devices hardly affect X-rays, ultrasound waves, radio frequency (RF) pulses, and/or any other forms of electromagnetic signals which may be used for capturing medical images. Thus, in conjunction with the development of wireless peripheral devices, a method for efficiently capturing medical images using the wireless peripheral devices is required.

### SUMMARY

Exemplary embodiments provide a medical image capturing apparatus and method for easily capturing medical images, which may be employed by a diagnostician in consideration of the remaining battery capacity of at least one peripheral device.

Exemplary embodiments also provide a medical image capturing apparatus and method for preventing interruption of the capturing of medical images of an object due to the battery discharge of a peripheral device when the medical images are captured using the medical image capturing apparatus.

According to an aspect of one or more exemplary embodiments, there is provided a method for capturing medical images of an object by using a medical image capturing apparatus, the method including: checking a remaining battery capacity of at least one peripheral device that is connected to the medical image capturing apparatus and that receives power from a battery; and providing information relating to at least one capturing scheme, which is performable by using the medical image capturing apparatus, from among a plurality of capturing schemes which are preset in the medical image capturing apparatus, which information is based on the checked remaining battery capacity.

The providing of the information relating to the at least one capturing scheme may include considering a respective battery consumption capacity with respect to each of the plurality of capturing schemes.

The medical image capturing apparatus may include a Magnetic Resonance Imaging (MRI) device, and the providing of the information relating to the at least one capturing scheme may further include considering a predicted charge capacity of the battery of the at least one peripheral device with respect to each of the plurality of capturing schemes.

The providing of the information relating to the at least one capturing scheme may include providing information relating to the at least one capturing scheme based on at least one part of the object.

The providing of the information relating to the at least one capturing scheme may include: receiving a selection of each of a first part and a second part of the object from a user; and providing information relating to a combination of a first capturing scheme for the first part and a second capturing scheme for the second part, each capturing scheme of which is performable by using the medical image capturing apparatus.

The providing of the information relating to at least one capturing scheme may include: receiving a selection of a first part of the object from a user; and providing information relating to at least another capturing scheme, which is performable by using the medical image capturing apparatus, based on a remaining battery capacity of a peripheral device which is used to photograph the first part.

The method may further include displaying the information relating to the at least one capturing scheme by discriminating the information from information relating to capturing schemes which are not performable by using the medical image capturing apparatus.

The method may further include displaying the checked remaining battery capacity of the at least one peripheral device by matching the checked remaining battery capacity with identification information relating to the at least one peripheral device.

The displaying may include: determining an operable time of the at least one peripheral device based on a battery consumption capacity with respect to a predetermined capturing scheme from among the plurality of capturing schemes; and displaying the determined operable time of the at least one peripheral device by matching the determined operable time with the identification information relating to the at least one peripheral device.

The method may further include: when a capturing scheme which is selected by a user from among the plurality of capturing schemes is a capturing scheme which is performable by using the medical image capturing apparatus, predicting a remaining battery capacity of the at least one peripheral device with respect to a case in which the medical images are captured by executing the selected capturing scheme; and providing information relating to at least another capturing scheme, which is performable by using the medical image capturing apparatus, from among the plurality of capturing schemes, based on a respective predicted remaining battery capacity.

The method may further include, when at least another capturing scheme which is selected by a user from among the plurality of capturing schemes is a capturing scheme which is not performable by using the medical image capturing apparatus, changing a parameter value included in the selected at least another capturing scheme based on the checked remaining battery capacity.

The changing of the parameter value may include displaying medical images to be captured by executing the capturing scheme in which the parameter value has been changed.

The parameter value may include at least one of a repetition time (TR), an echo time (TE), an imaging time, a matrix size, and a flip angle.

The plurality of capturing schemes may include at least one of a spin echo sequence and a gradient echo sequence.

The at least one peripheral device may include at least one of a radio frequency (RF) coil, an electrocardiogram (ECG) measurement device, a respiration measurement device, a body temperature measurement device, and a detector.

The checking of the remaining battery capacity of the at least one peripheral device may include receiving, by the medical image capturing apparatus, remaining battery capacity information from each of the at least one peripheral device.

According to another aspect of one or more exemplary embodiments, there is provided a method for capturing medical images of an object by using a medical image capturing apparatus which is configured for capturing medical images, the method including: determining a capturing scheme which is preset in the medical image capturing apparatus and identification information which relates to a peripheral device used in the determined capturing scheme; predicting a battery consumption capacity of the peripheral device based on the determined capturing scheme; and displaying the determined capturing scheme, the identification information which relates to the peripheral device used in the determined capturing scheme, and the predicted battery consumption capacity of the peripheral device based on the determined capturing scheme.

The medical image capturing apparatus may include a Magnetic Resonance Imaging (MRI) device, the predicting may include predicting a battery charge capacity of the peripheral device based on the determined capturing scheme, and the displaying may include displaying the predicted battery charge capacity.

According to another aspect of one or more exemplary embodiments, there is provided a method for capturing medical images of an object by using a medical image capturing apparatus which is configured for capturing medical images, the method including: checking a remaining battery capacity of at least one peripheral device which is connected to the medical image capturing apparatus while capturing medical images of the object based on a predetermined capturing scheme; and displaying a battery consumption capacity to be consumed thereafter by the at least one peripheral device based on the predetermined capturing scheme and the checked remaining battery capacity of the at least one peripheral device.

According to another aspect of one or more exemplary embodiments, there is provided a method for capturing medical images of an object by using a medical image capturing apparatus which is configured for capturing medical images, the method including: checking a remaining battery capacity of at least one peripheral device that is connected to the medical image capturing apparatus and that receives power from a battery, when a predetermined condition is satisfied; and providing information which relates to whether a capturing scheme which is preset in the medical image capturing apparatus is a capturing scheme which is performable by using the medical image capturing apparatus, based on the checked remaining battery capacity.

The medical image capturing apparatus may include at least one of a Magnetic Resonance Imaging (MRI) device and a computed tomography (CT) device, and the checking may include checking the remaining battery capacity of the at least one peripheral device when a table of the medical image capturing apparatus moves inside a gantry of the at least one of the MRI device and the CT device.

The checking may include checking the remaining battery capacity of the at least one peripheral device when information relating to the object is received by the medical image capturing apparatus from a user.

The checking may include checking the remaining battery capacity of the at least one peripheral device when a request relating to checking the remaining battery capacity of the at least one peripheral device is received by the medical image capturing apparatus from a user.

The medical image capturing apparatus may include at least one of an MRI device and a CT device, and the checking may include checking the remaining battery capacity of the at least one peripheral device when localizer imaging of the object is completed by the medical image capturing apparatus and a field of view (FoV) of the object is set.

The checking may include checking the remaining battery capacity of the at least one peripheral device when imaging of the object is completed based on a predetermined capturing scheme.

The preset capturing scheme may include a capturing scheme which is selected by the user, and the method may further include changing a parameter of the preset capturing scheme based on the checked remaining battery capacity of the at least one peripheral device when the preset capturing scheme is a capturing scheme which is not performable by using the medical image capturing apparatus.

According to another aspect of one or more exemplary embodiments, there is provided a non-transitory computer-readable recording medium which stores a computer-readable program for executing the method.

According to another aspect of one more exemplary embodiments, there is provided a medical image capturing apparatus for capturing medical images of an object, the apparatus including: a battery checking unit which is configured to check a remaining battery capacity of at least one peripheral device that is connected to the apparatus and that receives power from a battery; and a controller which is configured to provide information relating to at least one capturing scheme, which is performable by using the apparatus, from among a plurality of preset capturing schemes, which information is based on the checked remaining battery capacity.

The controller may be further configured to provide information with respect to the at least one capturing scheme based on considering a respective battery consumption capacity with respect to each of the plurality of capturing schemes.

The apparatus may further include a Magnetic Resonance Imaging (MRI) device, wherein the controller is further configured to provide information relating to the at least one capturing scheme based on considering a predicted charge capacity of the battery of the at least one peripheral device with respect to each of the plurality of capturing schemes.

The controller may be further configured to provide information relating to the at least one capturing scheme based on at least one part of the object.

The apparatus may further include a user input receiver which is configured to receive a selection of each of a first part and a second part of the object from a user, wherein the controller is further configured to provide information relating to a combination of a first capturing scheme for the first part and a second capturing scheme for the second part, each capturing scheme of which is performable by using the apparatus.

The apparatus may further include a user input receiver which is configured to receive a selection of a first part of the object from the user, wherein the controller is further configured to provide information relating to at least another capturing scheme, which is performable by using the apparatus, based on a remaining battery capacity of a peripheral device which is used to photograph the first part.

The apparatus may further include a display which is configured to display the information relating to the at least one capturing scheme by discriminating the information from information relating to capturing schemes which are not performable by using the apparatus.

The apparatus may further include a display which is configured to display the checked remaining battery capacity of the at least one peripheral device by matching the checked remaining battery capacity with identification information relating to the at least one peripheral device.

The controller may be further configured to determine an operable time of the at least one peripheral device based on a battery consumption capacity with respect to a predetermined capturing scheme from among the plurality of capturing schemes, and the display may be further configured to display the determined operable time of the at least one peripheral device by matching the determined operable time with the identification information relating to the at least one peripheral device.

When a capturing scheme which is selected by a user from among the plurality of capturing schemes is a capturing scheme which is performable by using the apparatus, the controller may be further configured to predict a remaining battery capacity of the at least one peripheral device with respect to a case in which the medical images are captured by executing the selected capturing scheme, and the controller may be further configured to provide information relating to at least another capturing scheme, which is performable by using the apparatus, from among the plurality of capturing schemes, based on the predicted remaining battery capacity.

When at least another capturing scheme which is selected by a user from among the plurality of capturing schemes is a capturing scheme which is not performable by using the apparatus, the controller may be further configured to change a parameter value included in the selected at least another capturing scheme based on the checked remaining battery capacity.

The apparatus may further include a display which is configured to display medical images to be captured by executing the capturing scheme in which the parameter value has been changed.

The parameter value may include at least one of a repetition time (TR), an echo time (TE), an imaging time, a matrix size, and a flip angle.

The plurality of capturing schemes may include at least one of a spin echo sequence and a gradient echo sequence.

The at least one peripheral device may include at least one of a radio frequency (RF) coil, an electrocardiogram (ECG) measurement device, a respiration measurement device, a body temperature measurement device, and a detector.

The battery checking unit may be further configured to receive remaining battery capacity information from the at least one peripheral device.

According to another aspect of one or more exemplary embodiments, there is provided a peripheral device that is connected to a medical image capturing apparatus which is configured to capture medical images of an object, the peripheral device including: a battery which is configured to supply power to the peripheral device; a battery information generator which is configured to generate remaining battery capacity information by checking a remaining battery capacity of the battery; and a communication unit which is configured to transmit the remaining battery capacity information to the medical image capturing apparatus.

The peripheral device may further include at least one of an electrocardiogram (ECG) measurement device which is configured to measure an ECG of the object, a respiration measurement device which is configured to measure a respiration of the object, a body temperature measurement device which is configured to measure a body temperature of the object, an X-ray receiver which is configured to receive X-rays which are emitted from the object, and a radio frequency (RF) signal receiver which is configured to receive RF signals which are emitted from the object.

The medical image capturing apparatus may include a Magnetic Resonance Imaging (MRI) device, and the peripheral device may further include a battery charger which is configured to charge the battery by using RF signals which are generated inside a gantry of the medical image capturing apparatus.

The communication unit may be further configured to transmit the remaining battery capacity information to the medical image capturing apparatus when a predetermined condition is satisfied.

### BRIEF DESCRIPTION OF THE DRAWINGS

The above and other features and advantages of the present inventive concept will become more apparent by describing in detail exemplary embodiments thereof with reference to the attached drawings in which:

FIG. 1 is a block diagram of a medical image capturing apparatus and peripheral devices which are connected to the medical image capturing apparatus, according to an exemplary embodiment;

FIG. 2 is a block diagram of a Magnetic Resonance Imaging (MRI) system;

FIG. 3A is a block diagram of a peripheral device which is connected to the medical image capturing apparatus, according to an exemplary embodiment, and FIG. 3B is a block diagram of a radio frequency (RF) coil which is connected to an MRI device, according to an exemplary embodiment;

FIG. 4 illustrates capturing schemes which are displayed on a display of the medical image capturing apparatus, according to an exemplary embodiment;

FIG. 5 illustrates capturing schemes which are displayed on the display of the medical image capturing apparatus, according to another exemplary embodiment;

FIG. 6 illustrates capturing schemes which are displayed on the display of the medical image capturing apparatus, according to another exemplary embodiment;

FIG. 7 illustrates remaining battery capacities of peripheral devices, which are displayed on the display of the medical image capturing apparatus, according to an exemplary embodiment;

FIG. 8 is a flowchart which illustrates a method for capturing medical images, according to an exemplary embodiment;

FIG. 9 is a flowchart which illustrates a method for capturing medical images, according to another exemplary embodiment;

FIG. 10 is a flowchart which illustrates a method for capturing medical images, according to another exemplary embodiment; and

FIG. 11 is a flowchart which illustrates a method for capturing medical images, according to another exemplary embodiment.

### DETAILED DESCRIPTION

Advantages and features of the exemplary embodiments and a method for achieving them will be clear with reference to the accompanying drawings, in which the exemplary embodiments are illustrated. The present inventive concept may, however, be embodied in many different forms and should not be construed as being limited to the exemplary embodiments set forth herein; rather, these exemplary embodiments are provided so that the present disclosure will be thorough and complete, and will fully convey the present disclosure to one of ordinary skill in the art. Like reference numerals can denote like elements throughout the specification.

The terms used in the specification will be schematically described, and the exemplary embodiments will be described in detail.

Although general terms as currently widely used as possible are selected as the terms used in the present disclosure while taking functions in the exemplary embodiments into account, they may vary based on an intention of one of ordinary skill in the art, judicial precedents, or the appearance of new technology. In addition, in specific cases, terms intentionally selected by the applicant may be used, and in this case, the meaning of the terms will be disclosed in a corresponding description of the exemplary embodiments. Accordingly, the terms used in the present disclosure should be defined not by simple names of the terms but by the meaning of the terms and the contents in the specification.

In the specification, when a certain part "includes" a certain component, this indicates that the part may further include another component instead of excluding another component unless there is different disclosure. In addition, the term "unit" as used with respect to the exemplary embodiments indicates a component which includes software or hardware, such as a Field Programmable Gate Array (FPGA) or an Application-Specific Integrated Circuit (ASIC), and the "unit" performs specified roles. However, the "unit" is not limited to software or hardware. The "unit" may be configured to be included in an addressable storage medium or to reproduce one or more processors. Therefore, for example, the "unit" may include at least one of components, such as software components, object-oriented software components, class components, and task components, processes, functions, attributes, procedures, subroutines, segments of program code, drivers, firmware, microcode, circuits, data, a database, data structures, tables, arrays, and variables. A function provided inside components and "units" may combine them into a smaller number of components and "units," or may further divide them into additional components and "units".

In the specification, the term "medical image capturing apparatus" indicates an apparatus which is configured for capturing medical images of an object and may include medical image capturing apparatuses having a scope which is obvious to one of ordinary skill in the art, such as an X-ray device, a computed tomography (CT) device, a magnetic resonance imaging (MRI) device, a positron emission tomography (PET) device, and the like. In addition, a "medical image capturing apparatus" may include at least one of a stationary medical image capturing apparatus and a mobile medical image capturing apparatus.

In the specification, the term "image" may indicate multi-dimensional data consisting of discrete image elements (e.g., pixels in a two-dimensional image or voxels in a three-dimensional image). For example, an "image" may include a medical image of an object, which is acquired by a CT device, an MRI device, an ultrasound device, or another medical image capturing apparatus.

In addition, in the specification, the term "object" may include a human being, an animal, or a part of the human being or the animal. For example, an "object" may include an organ, such as the liver, the heart, the womb, the brain, the breast, or the abdomen, or a blood vessel. In addition, an "object" may include a phantom. The phantom indicates a substance having a very approximate volume to a density and an effective atomic number of an organism. The phantom may include, for example, a spherical phantom which has similar properties to those of the human body.

In addition, in the specification, the term "user" may refer to a medical expert, such as a medical practitioner, a nurse, a medical laboratory technologist, a medical imaging expert, or the like, or may refer to a service engineer that repairs medical equipment, but "user" is not limited thereto.

In addition, in the specification, the term "capturing scheme" indicates a set of pre-stored parameter values of a medical image capturing apparatus. Capturing schemes which correspond to predetermined parts of an object or types of medical images may be preset by a manufacturing company of a medical image capturing apparatus or a user. The expression "capturing scheme" may be used interchangeably as the term "sequence" with respect to MRI devices and as the term "protocol" with respect to CT devices and X-ray devices.

In addition, in the specification, the term "parameter value" indicates an individual setting value of a medical image capturing apparatus which is configured for capturing medical images of an object.

In addition, in the specification, the term "peripheral device" indicates a device which is used to capture medical images of an object by being connected to a medical image capturing apparatus. Some peripheral devices may be mandatory to capture medical images of an object (e.g., a detector and an RF coil). The expression "peripheral device" may include a device without power lines (i.e., a wireless device) which is configured to receive power, and a device which is configured to receive power via a battery may be included in a peripheral device according to one or more exemplary embodiments even though the device is connected to a medical image capturing apparatus in a wired manner.

As used herein, the term "and/or" includes any and all combinations of one or more of the associated listed items. Expressions such as "at least one of," when preceding a list of elements, modify the entire list of elements and do not modify the individual elements of the list.

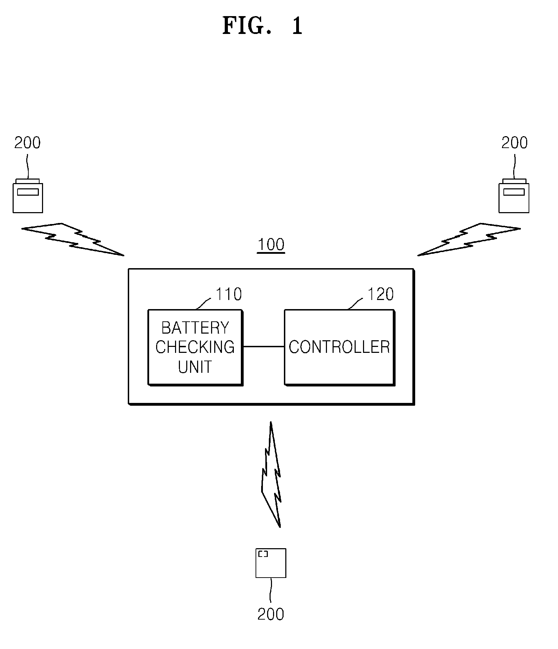

FIG. 1 is a block diagram of a medical image capturing apparatus 100 and at least one peripheral device 200 which is connected to the medical image capturing apparatus 100, according to an exemplary embodiment.

Referring to FIG. 1, the medical image capturing apparatus 100 may include a battery checking unit 110 and a controller 120. Either or both of the battery checking unit 110 and the controller 120 may include a microprocessor.

The at least one peripheral device 200 in Fig. 1 is wirelessly connected to the medical image capturing apparatus 100 and may wirelessly communicate with the medical image capturing apparatus 100 in accordance with a predetermined standard.

Alternatively, the at least one peripheral device 200 may include a device that is connected to the medical image capturing apparatus 100 in a wired manner and that receives power from a battery without power lines for receiving power.

The at least one peripheral device 200 may include at least one of an electrocardiogram (ECG) measurement device which is configured to measure an ECG of an object, a body temperature measurement device which is configured to measure a body temperature of the object, a radio frequency (RF) coil in a Magnetic Resonance Imaging (MRI) device, and a detector for an X-ray or CT device.

The battery checking unit 110 checks a remaining battery capacity of the at least one peripheral device 200 that is connected to the medical image capturing apparatus 100. The battery checking unit 110 may check a remaining battery capacity of each of the at least one peripheral device 200 as a ratio of a currently remaining battery capacity to a full battery capacity. The battery checking unit 110 may check the remaining battery capacity of the at least one peripheral device 200 by receiving respective remaining battery capacity information from each of the at least one peripheral device 200, or may directly measure the respective remaining battery capacity of the at least one peripheral device 200 by using any one or more of various well-known methods. For instance, remaining battery capacity of the at least one peripheral device 200 may be monitored by the battery checking unit 110 of the medical image capturing apparatus 100 on the basis of a signal strength of wireless communication from the peripheral device 200 to the medical image capturing apparatus 100. Such communication may relate to the image capturing process to be performed by the medical image capturing apparatus 100 in conjunction with the peripheral device 200.

The controller 120 controls components included in the medical image capturing apparatus 100 and may provide information relating to at least one capturing scheme, which is performable by using the medical image capturing apparatus 100, from among a plurality of capturing schemes preset in the medical image capturing apparatus 100, which information is based on the remaining battery capacity of the at least one peripheral device 200.

The controller 120 of the medical image capturing apparatus 100 may indicate to user only the at least one capturing scheme, which is performable by using the medical image capturing apparatus 100, from among a plurality of capturing schemes preset in the medical image capturing apparatus 100. Alternatively, the controller may indicate a full list of all available schemes of the plurality of capturing schemes preset in the medical image capturing apparatus 100, and highlight capturing schemes which can be completed before a remaining battery charge of the peripheral device runs out, or attenuate display of schemes that can not be concluded before remaining battery charge runs out.

In addition, when a predetermined capturing scheme is selected by a user, the controller 120 may provide information which relates to whether the capturing scheme selected by the user is a capturing scheme which is performable by using the medical image capturing apparatus 100, based on the checked remaining battery capacity of the at least one peripheral device 200.

The at least one capturing scheme, which is performable by using the medical image capturing apparatus 100, indicates a capturing scheme by which medical images can be stably captured without or before full discharge of a battery of the at least one peripheral device 200 while the medical image capturing apparatus 100 is capturing the medical images. That is to say that the process of image capturing must be able to be completed on a remaining charge of a battery of the peripheral device, and before the battery becomes depleted of charge.

Capturing schemes may include at least one of a spin echo sequence, a gradient echo sequence, a susceptibility weighted imaging sequence, and the like with respect to an MRI device, a brain protocol, a spine protocol, a blood vessel protocol, and the like with respect to a CT device, and a chest protocol, a spine protocol, and the like with respect to an X-ray device, but the capturing schemes are not limited thereto. It is noted in this respect, that power consumption by a peripheral device is not only determined by the power needed for communications by the peripheral device with the medical image capturing apparatus 100, and that some capturing schemes may require a longer time or a shorter time to complete, or involve elements or components that draw less or more power from the battery. Consequently, individual capturing schemes may exhibits distinct power consumption needs.

The controller 120 may provide information which relates to a capturing scheme, which is performable by using the medical image capturing apparatus 100, by considering a respective battery consumption capacity with respect to each of the plurality of capturing schemes and a remaining battery capacity of the at least one peripheral device 200. In detail, the controller 120 may determine a capturing scheme requiring a battery consumption capacity that is less than the remaining battery capacity of the at least one peripheral device 200 as a capturing scheme to be executed, and may provide information relating to the determined capturing scheme.

When the medical image capturing apparatus 100 includes an MRI device, the at least one peripheral device 200 which is connected to the MRI device may charge the battery by using RF signals which are generated inside a gantry, and in this case, the controller 120 may provide information which relates to a capturing scheme, which is performable by using the medical image capturing apparatus 100, by further considering a predicted charge capacity of the at least one peripheral device 200 in addition to the remaining battery capacity of the at least one peripheral device 200 and the respective battery consumption capacity with respect to each of the plurality of capturing schemes.

In detail, the controller 120 may determine a capturing scheme which requires a battery consumption capacity that is less than a total sum of the predicted charge capacity of the at least one peripheral device 200 and the remaining battery capacity of the at least one peripheral device 200 based on each of the plurality of capturing schemes as a capturing scheme to be executed, and may provide information which relates to the determined capturing scheme.

Although not shown in FIG. 1, the medical image capturing apparatus 100 according to another exemplary embodiment may further include a display, wherein the controller 120 controls the display to display the information relating to the at least one capturing scheme. The information relating to a particular capturing scheme may include an identification (ID) of the particular capturing scheme in order to specify the particular capturing scheme.

The medical image capturing apparatus 100 according to an exemplary embodiment may include a user input receiver which is configured to receive, from a user, a selection of at least one part for which medical images are to be captured of the object. The user may select a part for which medical images are to be captured by using any one or more of various methods and/or devices, including a keyboard, a mouse, a trackball, a touch screen, and/or the like.

When the user input receiver receives a selection of a first part of the object from the user, the controller 120 may provide information relating to a capturing scheme, which is performable by using the medical image capturing apparatus 100, by considering only a remaining battery capacity of a peripheral device 200 which is being used to photograph the first part from among the at least one peripheral device 200.

For example, because an MRI device may include a respective RF coil for each part of several corresponding parts of the object, when the user selects the abdomen of the object as a part for which medical images are to be captured, the controller 120 may consider only a remaining battery capacity of an abdominal RF coil and a remaining battery capacity of peripheral devices 200 required to photograph the abdomen in providing information relating to a particular capturing scheme, thereby effectively excluding a provision of information relating to respective capturing sequences which relate respectively to each of a chest RF coil and a head RF coil.

A time point at which the battery checking unit 110 checks a remaining battery capacity of the at least one peripheral device 200 may be variably set. For example, the battery checking unit 110 may check a remaining battery capacity of the at least one peripheral device 200 if and/or when a predetermined condition is satisfied. At least one such predetermined condition may apply, amongst which the following examples are provided below.

First, if the medical image capturing apparatus 100 includes an MRI or CT device, the battery checking unit 110 may check a remaining battery capacity of the at least one peripheral device 200 when a table of the medical image capturing apparatus 100 moves inside a gantry of the MRI or CT device. In particular, when an object moves inside the gantry of the MRI or CT device, the controller 120 may provide information which relates to at least one capturing scheme, which is performable by using the medical image capturing apparatus 100, from among the plurality of capturing schemes, and/or information which relates to whether a capturing scheme selected by the user is a capturing scheme which is performable by using the medical image capturing apparatus 100.

Second, when the user of the medical image capturing apparatus 100 inputs information which relates to the object to the medical image capturing apparatus 100, the battery checking unit 110 may check a remaining battery capacity of the at least one peripheral device 200. The information which relates to the object may include at least one of a name, sex, age, and/or a body condition for identifying the object. This enables a more fine assessment based on the object, whether the capturing can be completed on a remaining charge of a peripheral device.

Third, when the user inputs a request which relates to checking a remaining battery capacity of the at least one peripheral device 200 to the medical image capturing apparatus 100, the battery checking unit 110 may check a remaining battery capacity of the at least one peripheral device 200. As a result, the user may easily determine which peripheral device 200 requires a battery replacement.

Fourth, if the medical image capturing apparatus 100 includes an MRI or CT device, the battery checking unit 110 may check a remaining battery capacity of the at least one peripheral device 200 when localizer capturing of the object is completed by the medical image capturing apparatus 100 and a field of view (FoV) of the object is set. Thus the actual extent of the capturing process may be taken into account to determine if the peripheral device has sufficient charge remaining to complete the intended capturing scheme.

Fifth, when imaging of the object is completed based on a predetermined capturing scheme, the battery checking unit 110 may check a remaining battery capacity of the at least one peripheral device 200. As a result, the user may easily determine which peripheral device 200 has a relatively low remaining battery capacity after the capturing is completed. The battery may then be replaced, for example for recharging, to enable a new capturing process to be initiated with fully charged batteries of the relevant peripheral device.

Sixth, the battery checking unit 110 may check a remaining battery capacity of the at least one peripheral device 200 while the medical image capturing apparatus 100 is capturing medical images by executing a predetermined capturing scheme. In this case, the controller 120 may provide, to the user, information relating to the remaining battery capacity and a battery consumption capacity to be thereafter consumed by the at least one peripheral device 200 based on the predetermined capturing scheme. In this case, the user may prevent continuation of imaging of the object in a fully discharged state of a peripheral device 200 by checking a remaining battery capacity of the at least one peripheral device 200 and quickly stopping the imaging of the object prior to a time when a battery of the peripheral device 200 is predicted to be fully discharged. Thus at least a partial imaging may be completed.

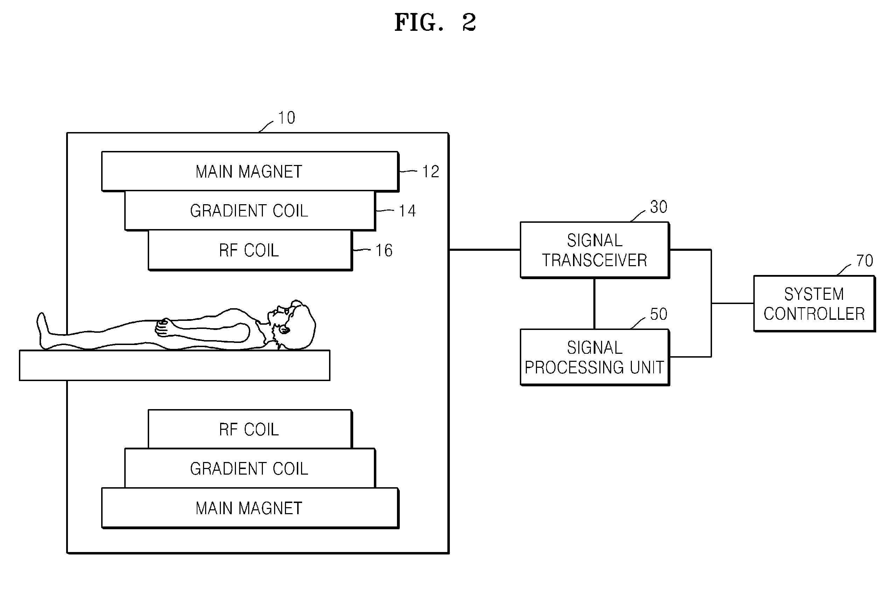

The medical image capturing apparatus 100 according to an exemplary embodiment may be included in an MRI system to be described with reference to FIG. 2.

FIG. 2 is a block diagram of an MRI system. Referring to FIG. 2, the MRI system may include a gantry 10, a signal transceiver 30, a signal processor 50, and a system controller 70.

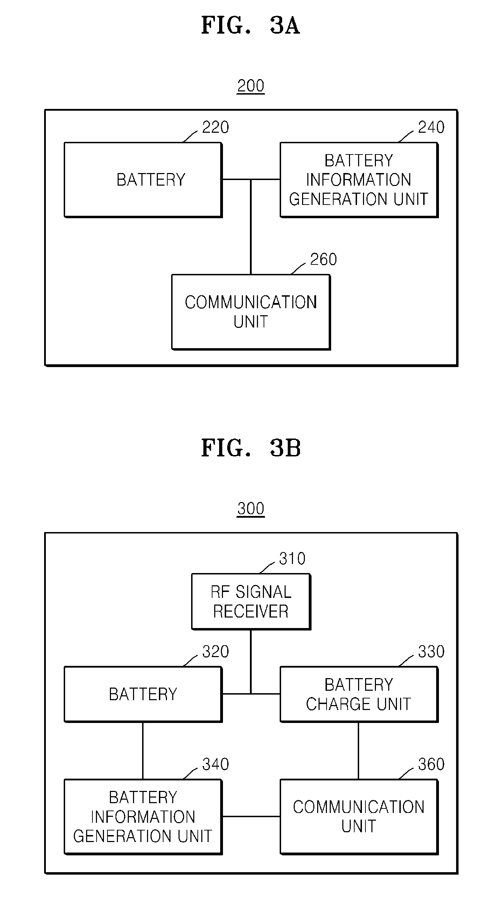

The gantry 10 may be located in a shielded room in order to block electromagnetic waves generated by a main magnet 12, a gradient coil 14, and an RF coil 16 from being radiated to the outside. In the gantry 10, a static magnetic field and a gradient magnetic field are formed, and RF signals may be irradiated toward an object. As indicated above and below in relation to Fig. 3B, the RF coil may be wireless. This may encompass wireless communications of the RF coil 16 with the signal transceiver 30, or wired communications, and the RF coil 16 is battery powered.

The gantry 10 may include the main magnet 12, the gradient coil 14, and the RF coil 16.

The main magnet 12, the gradient coil 14, and the RF coil 16 may be disposed along a predetermined direction inside the gantry 10. The predetermined direction may include, for example, a coaxial cylindrical direction and the like. For example, the main magnet 12, the gradient coil 14, and the RF coil 16 may be disposed along a coaxial cylinder. An object may be placed on a table, which can be inserted into the coaxial cylinder along a horizontal axis of the coaxial cylinder.

The main magnet 12 generates a static magnetic field which aligns directions of magnetic dipole moments of atomic nuclei of the object in a predetermined direction.

The gradient coil 14 includes X, Y, and Z coils which respectively generate gradient magnetic fields in each of an X-axis direction, a Y-axis direction, and a Z-axis direction that are mutually orthogonal to each other. The gradient coil 14 may provide location information which relates to each part of the object by variably inducing a respective resonance frequency for each respective part of the object.

The RF coil 16 may apply RF signals to a patient (the object) and receive RF signals which are emitted from the patient. In detail, the RF coil 16 may transmit RF pulses which have a same frequency as a frequency of precession by atomic nuclei of the patient, and then may stop the transmission of the RF signals and receive RF signals which are emitted from the patient as a result of the transmission of the pulses.

The signal transceiver 30 may control a gradient magnetic field which is formed inside the gantry 10 by executing a predetermined capturing scheme and thereby control the transmission and reception of the RF signals.

The signal processor 50 may generate MRI data which relates to the object by processing the RF signals received by the signal transceiver 30.

The system controller 70 controls operations of the signal transceiver 30 and the signal processor 50. The system controller 70 may control a capturing scheme which is implemented by the signal transceiver 30, and may also control a processing of the RF signals which is implemented by the signal processor 50.

FIG. 3A is a block diagram of a peripheral device 200 which is connected to the medical image capturing apparatus 100, according to an exemplary embodiment, and FIG. 3B is a block diagram of an RF coil 300 which is connected to an MRI device, according to an exemplary embodiment.

Referring to FIG. 3A, the peripheral device 200 may include a battery 220, a battery information generator 240, and a communication unit 260.

Although not shown in FIG. 3A, the peripheral device 200 which is connected to the medical image capturing apparatus 100, according to an exemplary embodiment, may further include at least one of an ECG measurement device which is configured to measure an ECG of an object, a respiration measurement device which is configured to measure a respiration of the object, a body temperature measurement device which is configured to measure a body temperature of the object, an X-ray receiver which is configured to receive X-rays which are emitted from the object, and an RF signal receiver which is configured to receive RF signals which are emitted from the object.

The battery 220 supplies power to elements, circuits, and other portions which are included in the peripheral device 200 in order to drive the peripheral device 200. The battery information generator 240 checks a remaining capacity of the battery 220 and generates remaining battery capacity information to be transmitted to the medical image capturing apparatus 100.

The communication unit 260 performs wired and/or wireless communication with the medical image capturing apparatus 100 and transmits the remaining battery capacity information and identification information which relates to the peripheral device 200 to the medical image capturing apparatus 100. The identification information which relates to the peripheral device 200 may be stored in a memory unit (not shown) of the peripheral device 200 or received from an external device.

A time point at which the peripheral device 200 transmits the remaining battery capacity information and the identification information which relates to the peripheral device 200 to the medical image capturing apparatus 100 may be variably set. For example, if a predetermined condition is satisfied, the peripheral device 200 may transmit the remaining battery capacity information and the identification information relating thereto to the medical image capturing apparatus 100.

A time point or a transmission condition by which the peripheral device 200 is configured to transmit the remaining battery capacity information and the identification information relating thereto to the medical image capturing apparatus 100 may be the same as a remaining battery capacity checking time point or a transmission condition which relates to the battery checking unit 110 described above.

FIG. 3B is a block diagram of the RF coil 300 of an MRI device, according to an exemplary embodiment. The RF coil 300 of FIG. 3B may be included in the peripheral device 200, according to an exemplary embodiment.

Referring to FIG. 3B, the RF coil 300 may include an RF signal receiver 310, a battery 320, a battery charger 330, a battery information generator 340, and a communication unit 360. Since descriptions of the battery information generator 340 and the communication unit 360 correspond to respective ones described above with respect to FIG. 3A, a detailed description thereof is omitted.

The RF signal receiver 310 receives RF signals which are emitted from an object. The RF signal receiver 310 may be configured to receive only the RF signals which are emitted from the object and/or configured to not only receive the RF signals but also transmit RF signals.

The battery 320 supplies power to elements, circuits, and other portions which are included in the RF coil 300 in order to drive the RF coil 300. The battery charger 330 charges the battery 320 by using RF signals which are generated inside a gantry. The battery charger is only set in operation to charge the battery 320, when the RF coil 300 is not performing a capturing scheme and the MRI device is at rest, i.e. not being used to create images of an object. In this manner the battery may be charged at times, during which the MRI device is inoperative, so as not to create any disturbance or interference or the like, resulting from for instance AC power supply, even if supplied through a rectifier (not shown), for charging the battery 320. When multiple capturing schemes are performed successively, the intermediate times may be insufficient to fully charge the battery to its full capacity. Consequently, at any given remaining charge of the battery 320, a situation may occur at which not all capturing schemes, which the MRI device is in principle capable of performing, may be available to be performed due to insufficient remaining charge of the battery.

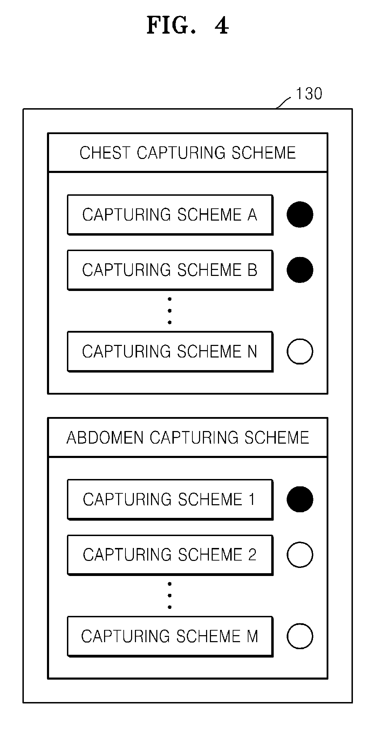

Referring back to FIG. 1, the controller 120 may provide respective information which relates to a respective capturing scheme, which is performable by using the medical image capturing apparatus 100, for each part of the object. This will now be described with reference to FIG. 4.

FIG. 4 illustrates capturing schemes which are displayed on a display 130 of the medical image capturing apparatus 100, according to an exemplary embodiment.

As shown in FIG. 4, the display 130 may display information which relates to respective capturing schemes, which are performable by using the medical image capturing apparatus 100, for example at least one scheme for each part or a whole of an object.

Referring to FIG. 4, capturing schemes A, B, ..., N are chest capturing schemes, and capturing schemes 1, 2, ..., M are abdomen capturing schemes, wherein a black circle is displayed on the right side of each of the capturing schemes A, B, and 1, which are performable by using the medical image capturing apparatus 100, and an empty circle is displayed on the right side of each of the capturing schemes N, 2, and M, which are not performable by using the medical image capturing apparatus 100. In particular, the display 130 may display information which relates to capturing schemes which are performable by using the medical image capturing apparatus 100 by discriminating the information from information which relates to capturing schemes which are not performable by using the medical image capturing apparatus 100. Any one or more of various well-known methods, in addition to the method shown in FIG. 4, may be used as a capturing scheme discriminating method.

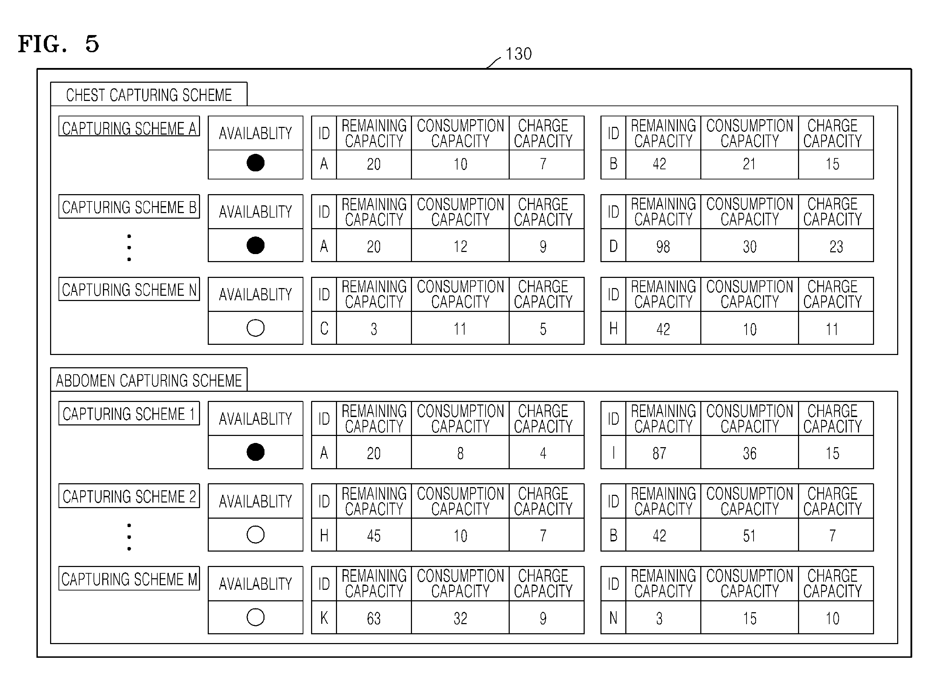

FIG. 5 illustrates capturing schemes which are displayed on the display 130 of the medical image capturing apparatus 100, according to another exemplary embodiment.

Referring to FIG. 5, the display 130 allows a user to easily determine which peripheral device 200 requires a battery replacement by providing not only information which relates to capturing schemes which are performable by using the medical image capturing apparatus 100, but also information which relates to each of a remaining battery capacity, a battery consumption capacity with respect to each capturing scheme, and a respective predicted charge capacity of each of the peripheral devices 200 used or necessary for each capturing scheme.

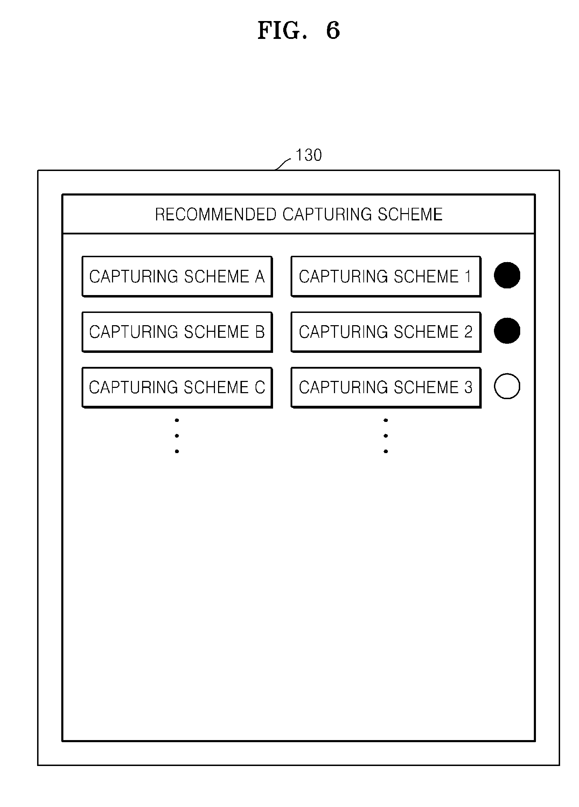

FIG. 6 illustrates a plurality of capturing schemes which are displayed on the display 130 of the medical image capturing apparatus 100, according to another exemplary embodiment.

Referring to FIG. 6, the display 130 displays information which relates to combinations of the chest capturing schemes and the abdomen capturing schemes shown in FIG. 4.

When the user input receiver receives, from a user, a selection of two or more parts of the object, the controller 120 may provide information which relates to a combination of capturing schemes for each of the parts selected by the user.

For example, if the user selects the abdomen and the chest of the object as parts for which medical images are to be captured, the controller 120 calculates a sum of a battery consumption capacity with respect to a first capturing scheme from among abdomen capturing schemes and a battery consumption capacity with respect to a second capturing scheme from among chest capturing schemes. If the sum of the battery consumption capacities is less than a remaining battery capacity of a peripheral device 200 to be used, the controller 120 may provide information for display on the display 130 which relates to a combination of the first capturing scheme and the second capturing scheme as a capturing scheme set, which is performable by using the medical image capturing apparatus 100. In FIG. 6, a first combination of a capturing scheme A and a capturing scheme 1 and a second combination of a capturing scheme B and a capturing scheme 2 are displayed as capturing scheme sets which are performable by using the medical image capturing apparatus 100, as indicated by the corresponding black circles, and a third combination of a capturing scheme C and a capturing scheme 3 is displayed as a capturing scheme set which is not performable by using the medical image capturing apparatus 100, as indicated by the corresponding empty circle.

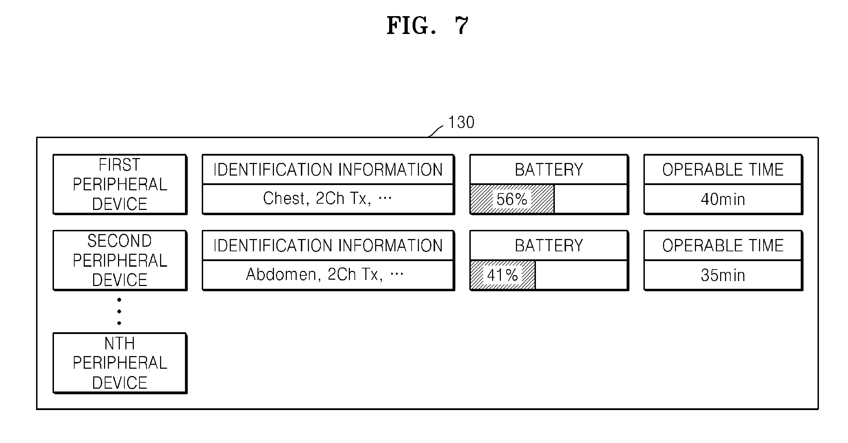

FIG. 7 illustrates remaining battery capacities of peripheral devices 200, which are displayed on the display 130 of the medical image capturing apparatus 100, according to an exemplary embodiment.

Referring to FIG. 7, the display 130 may display identification information and a remaining battery capacity of each of the at least one peripheral device 200. The controller 120 may control the battery checking unit 110 and the display 130 to display remaining battery capacities of peripheral devices 200, which are checked by the battery checking unit 110, on the display 130.

The identification information shown in FIG. 7 is information which specifies each of the at least one peripheral device 200 and may include a respective part of the object for which corresponding peripheral devices 200 are used, IDs of the peripheral devices 200, and/or any other relevant information.
The display 130 may display remaining battery capacity information as a ratio of a remaining battery capacity to a full battery capacity, and may display a respective operable time of each of the at least one peripheral device 200. The operable time of each of the at least one peripheral device 200 indicates an operable time of each of the at least one peripheral device 200 during which medical images are captured in conjunction with an execution of a predetermined capturing scheme, based on a battery consumption capacity of the predetermined capturing scheme from among the plurality of capturing schemes. The predetermined capturing scheme may include a capturing scheme which corresponds to the highest battery consumption capacity from among the plurality of capturing schemes.

The display 130 may display a remaining battery capacity and/or an operable time of each of the at least one peripheral device 200 by matching the checked remaining battery capacity and/or the determined operable time with identification information which relates to the corresponding peripheral device 200.

The user may select any one of a plurality of capturing schemes, wherein the capturing scheme selected by the user may be a capturing scheme which may or may not be performable by using the medical image capturing apparatus 100.

When the capturing scheme selected by the user is a capturing scheme which is performable by using the medical image capturing apparatus 100, the controller 120 predicts a remaining battery capacity of at least one peripheral device 200 to be used when medical images are captured by executing the selected capturing scheme. For example, if a remaining battery capacity of a first peripheral device 200 is 10, and a battery consumption capacity of the capturing scheme selected by the user is 5, the controller 120 predicts a remaining battery capacity of the first peripheral device 200 as being equal to 10 - 5 = 5 in a case in which medical images, based on the capturing scheme selected by the user, are completely captured. Thereafter, the controller 120 provides information which relates to capturing schemes which are performable by using the medical image capturing apparatus 100 based on the remaining battery capacity of 5. Thusly, the medical image capturing apparatus 100 captures medical images of the object by executing capturing schemes selected by the user.

According to another exemplary embodiment, the controller 120 may capture medical images of the object by executing a capturing scheme selected by the user, recheck a remaining battery capacity of each of the at least one peripheral devices 200, and provide information which relates to capturing schemes which are performable by using the medical image capturing apparatus 100. This allows information to the user, on particular is an actual power consumption exceeds an expected power consumption of the peripheral device.

When the capturing scheme selected by the user is a capturing scheme which is not performable by using the medical image capturing apparatus 100, the controller 120 may change a parameter value which is included in the capturing scheme selected by the user based on the remaining battery capacity checked by the battery checking unit 110. The parameter value may include at least one of a repetition time (TR), an echo time (TE), an imaging time, a matrix size, a flip angle, a tube voltage, and a tube current.

For example, if a remaining battery capacity of the first peripheral device 200 is 10, and a battery consumption capacity which corresponds to the capturing scheme selected by the user is 13, the controller 120 may change a parameter value included in the capturing scheme selected by the user so that the battery consumption capacity which corresponds to the capturing scheme selected by the user is less than 10. The controller 120 may decrease the battery consumption capacity of the capturing scheme selected by the user by changing the parameter value included in the capturing scheme selected by the user. It should be acknowledged that this entails a risk of decreasing the quality of medical images, but for predetermined cases, such as very urgent ones, such a loss in medical image quality may be acceptable, when compared with time losses to replace or recharge a battery.

Because the user may not be satisfied with the quality of medical images when medical images are acquired in conjunction with an execution of the capturing scheme of which the parameter value has been changed, the controller 120 may provide a preview image to the user by displaying medical images to be captured by executing the capturing scheme of which the parameter value has been changed on the display. The user may then directly change a parameter value and even overrule in comparison with the parameter values changed by the controller. The preview allows a user to determine whether an obtainable image quality may suffice for any intended use, or whether the parameters need to be adapted further, even reverted to default values.

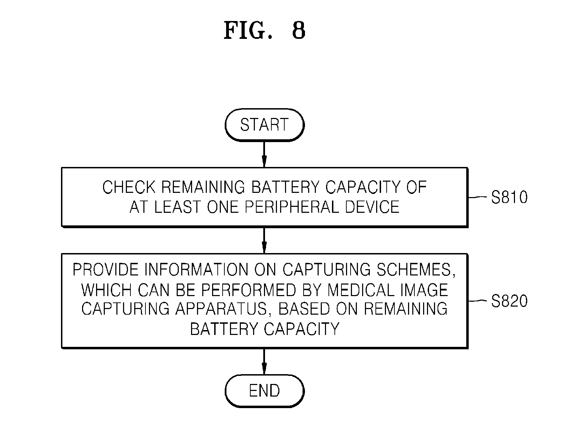

FIG. 8 is a flowchart which illustrates a method for capturing medical images, according to an exemplary embodiment. Referring to FIG. 8, the method includes operations sequentially processed by the medical image capturing apparatus 100 shown in FIG. 1. Thus, although omitted hereinafter, the above descriptions of the medical image capturing apparatus 100 shown in FIG. 1 also apply to the method of FIG. 8.

In operation S810, the medical image capturing apparatus 100 checks a remaining battery capacity of the at least one peripheral device 200 that is connected thereto and that receives power from a battery. The medical image capturing apparatus 100 may receive remaining battery capacity information from each of the at least one peripheral device 200, or may directly measure a remaining battery capacity of each of the at least one peripheral device 200.

In operation S820, the medical image capturing apparatus 100 provides information which relates to capturing schemes, which are performable by using the medical image capturing apparatus 100, from among a plurality of preset capturing schemes, based on a remaining battery capacity of each of the at least one peripheral device 200. Alternatively, the medical image capturing apparatus 100 may provide information which relates to whether a predetermined capturing scheme selected by a user is a capturing scheme which is performable by using the medical image capturing apparatus 100, based on a remaining battery capacity of each of the at least one peripheral device 200.

The medical image capturing apparatus 100 may provide the information which relates to capturing schemes by considering a battery consumption capacity of each of a plurality of capturing schemes. In addition, when the medical image capturing apparatus 100 includes an MRI device, the medical image capturing apparatus 100 may provide the information which relates to capturing schemes by further considering a respective predicted charge capacity of each of corresponding batteries of peripheral devices 200 based on each of the plurality of capturing schemes. The controller may be adapted to allow display only of performable schemes, or indicate which of all the preset available schemes are performable (can be performed) in view of an actual charge of the battery and any one of predicted resulting charge, power consumption during execution of the capturing scheme, or the like.

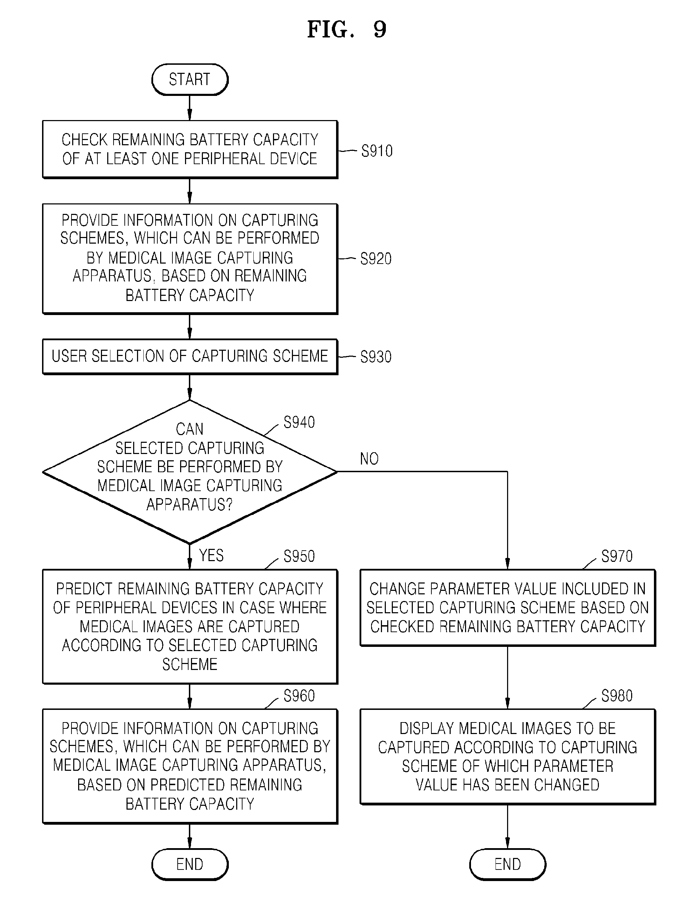

FIG. 9 is a flowchart which illustrates a method for capturing medical images, according to another exemplary embodiment.

Since operations S910 and S920 are identical to operations S810 and S820 of FIG. 8, a detailed description thereof is omitted.

In operation S930, the medical image capturing apparatus 100 receives, from a user, a selection of an arbitrary capturing scheme. The user may select a capturing scheme which may or may not be performable by using the medical image capturing apparatus 100.

In operation S940, the medical image capturing apparatus 100 determines whether the capturing scheme selected by the user is a capturing scheme which is performable by using the medical image capturing apparatus 100.

If the capturing scheme selected by the user is determined to be a capturing scheme which is performable by using the medical image capturing apparatus 100, the medical image capturing apparatus 100 predicts a respective remaining battery capacity of each of peripheral devices 200 to be used when medical images are captured in conjunction with an execution of the capturing scheme selected by the user, in operation S950.

In operation S960, the medical image capturing apparatus 100 provides information which relates to capturing schemes, which are performable by using the medical image capturing apparatus 100, based on the respective predicted remaining battery capacity. In particular, when an arbitrary capturing scheme is selected by the user, the medical image capturing apparatus 100 may update information which relates capturing schemes which are performable by using the medical image capturing apparatus 100.

If it is determined in operation S940 that the capturing scheme selected by the user is a capturing scheme which is not performable by using the medical image capturing apparatus 100, the medical image capturing apparatus 100 changes a parameter value included in the capturing scheme selected by the user based on a remaining battery capacity of the at least one wireless battery 200 in operation S970. In detail, the medical image capturing apparatus 100 changes the parameter value so that a battery consumption capacity which corresponds to the capturing scheme selected by the user is included in the remaining battery capacity of the at least one wireless battery 200.

In operation S980, the medical image capturing apparatus 100 displays medical images to be captured in conjunction with an execution of the capturing scheme of which the parameter value has been changed. The user may check a preview image of the medical images to be captured in conjunction with an execution of the capturing scheme of which the parameter value has been changed in order to determine whether the medical images are captured by executing the capturing scheme of which the parameter value has been changed.

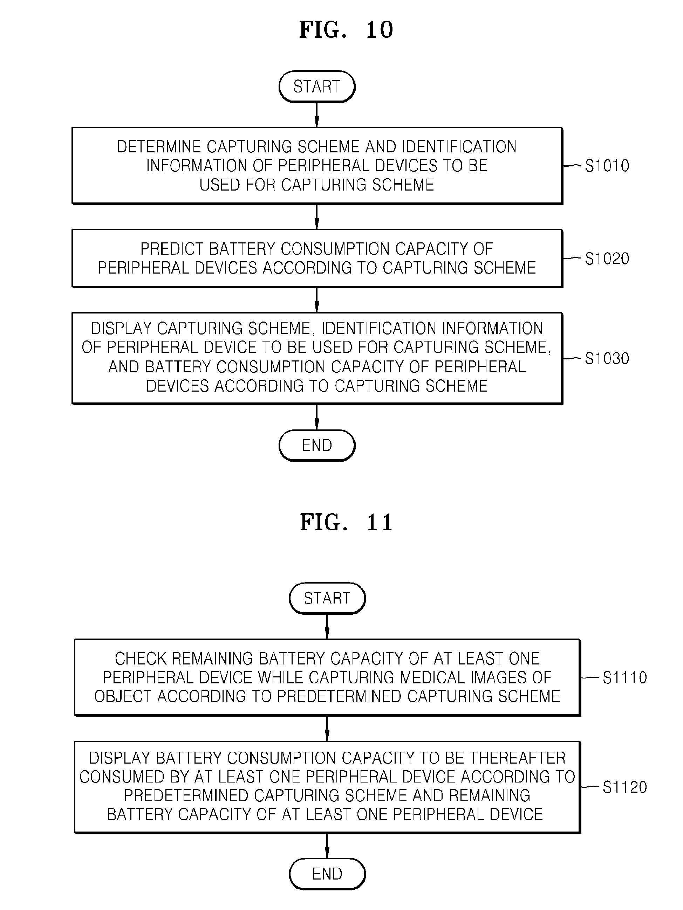

FIG. 10 is a flowchart which illustrates a method for capturing medical images, according to another exemplary embodiment.

In operation S1010, the medical image capturing apparatus 100 determines a capturing scheme preset therein and identification information which relates to peripheral devices 200 to be used in conjunction with an execution of the capturing scheme. The preset capturing scheme may be a capturing scheme which is selected by a user.

In operation S1020, the medical image capturing apparatus 100 predicts a respective battery consumption capacity of each of the peripheral devices 200 based on the determined capturing scheme. When the medical image capturing apparatus 100 includes an MRI device, the medical image capturing apparatus 100 may predict a respective battery charge capacity of each of the peripheral devices 200 based on the determined capturing scheme.

In operation S1030, the medical image capturing apparatus 100 displays the capturing scheme, the identification information which relates to each of the peripheral devices 200 to be used for the capturing scheme, and the respective battery consumption capacity of each of the peripheral devices 200 based on the determined capturing scheme. When the medical image capturing apparatus 100 includes an MRI device, the medical image capturing apparatus 100 may further display the respective predicted battery charge capacity of each of the peripheral devices 200 based on the determined capturing scheme.

FIG. 11 is a flowchart which illustrates a method for capturing medical images, according to another exemplary embodiment.

In operation S1110, the medical image capturing apparatus 100 checks a remaining battery capacity of at least one peripheral device 200 which is connected to the medical image capturing apparatus 100 while capturing medical images of an object during an execution of a predetermined capturing scheme.

In operation S1120, the medical image capturing apparatus 100 displays the remaining battery capacity of the at least one peripheral device 200 and a battery consumption capacity to be thereafter consumed by the at least one peripheral device 200 in conjunction with an execution of the predetermined capturing scheme.

Exemplary embodiments can be written as computer programs and/or implemented in general-use digital computers that execute the programs using a non-transitory or transitory computer-readable recording medium.

While exemplary embodiments have been described above with reference to the accompanying drawings, it will be understood by one of ordinary skill in the art that various changes in form and details may be made therein without changing the technical spirit and mandatory features of the present inventive concept according to the appended claims. Therefore, the exemplary embodiments should be understood in the illustrative sense only and not for the purpose of any limitation on an interpretation of the scope of protection according to the appended claims in any respect.

## Claims

1. A method for capturing medical images of at least a part of an object by using a medical image capturing apparatus, the method comprising:
checking a remaining battery capacity of at least one peripheral device that is connected to the medical image capturing apparatus and that receives power from a battery; and
providing information relating to at least one capturing scheme, which is performable by using the medical image capturing apparatus, from among a plurality of capturing schemes which are preset in the medical image capturing apparatus, which information is based on the checked remaining battery capacity.

2. The method of claim 1, wherein the providing of the information relating to the at least one capturing scheme is further based on a respective battery consumption capacity with respect to each of the plurality of capturing schemes.

3. The method of claim 1 or 2, wherein the medical image capturing apparatus comprises a Magnetic Resonance Imaging (MRI) device, and
the providing of the information relating to the at least one capturing scheme further comprises considering a predicted charge capacity of a battery of the at least one peripheral device with respect to each of the plurality of capturing schemes.

4. The method of claim 1, 2 or 3, wherein the providing of the information relating to the at least one capturing scheme comprises providing information relating to the at least one capturing scheme based on at least one part of the object.

5. The method of any of the preceding claims, wherein the providing of the information relating to the at least one capturing scheme comprises:
receiving a selection of each of a first part and a second part of the object from a user; and
providing information relating to a combination of a first capturing scheme for the first part and a second capturing scheme for the second part, each capturing scheme of which is performable by using the medical image capturing apparatus.

6. The method of any of the preceding claims, wherein the providing of the information relating to the at least one capturing scheme comprises:
receiving a selection of a first part of the object from a user; and
providing information relating to the at least one capturing scheme, which is performable by using the medical image capturing apparatus, by considering a remaining battery capacity of a peripheral device which is used to photograph the first part.

7. The method of any of the preceding claims, further comprising displaying the checked remaining battery capacity of the at least one peripheral device by matching the checked remaining battery capacity with identification information relating to the at least one peripheral device.

8. The method of any of the preceding claims, wherein the displaying comprises:
determining an operable time of the at least one peripheral device based on a battery consumption capacity with respect to a predetermined capturing scheme from among the plurality of capturing schemes; and
displaying the determined operable time of the at least one peripheral device by matching the determined operable time with the identification information relating to the at least one peripheral device.

9. The method of any of the preceding claims, further comprising:
when a capturing scheme which is selected by a user from among the plurality of capturing schemes is a capturing scheme, which is performable by using the medical image capturing apparatus, predicting a remaining battery capacity of the at least one peripheral device with respect to a case in which the medical images are captured by executing the selected capturing scheme; and
providing information relating to at least another capturing scheme, which is performable by using the medical image capturing apparatus, from among the plurality of capturing schemes, based on a respective predicted remaining battery capacity.

10. The method of any of the preceding claims, further comprising, when a capturing scheme which is selected by a user from among the plurality of capturing schemes is a capturing scheme which is not performable by using the medical image capturing apparatus, changing a parameter value included in the selected capturing scheme based on the checked remaining battery capacity.

11. The method of claim 10, wherein the changing the parameter value comprises displaying medical images to be captured by executing the capturing scheme in which the parameter value has been changed.

12. A non-transitory computer-readable recording medium which stores a computer-readable program for executing the method of any one of claims 1 through 11.

13. A medical image capturing apparatus for capturing medical images of an object, the apparatus comprising:
a battery checking unit which is configured to check a remaining battery capacity of at least one peripheral device that is connected to the apparatus and that receives power from a battery; and
a controller which is configured to provide information relating to at least one capturing scheme, which is performable by using the apparatus, from among a plurality of preset capturing schemes, which information is based on the checked remaining battery capacity.

14. A peripheral device that is connected to a medical image capturing apparatus which is configured to capture medical images of an object, the peripheral device comprising:
a battery which is configured to supply power to the peripheral device;
a battery information generator which is configured to generate remaining battery capacity information by checking a remaining battery capacity of the battery; and
a communication unit which is configured to transmit the generated remaining battery capacity information to the medical image capturing apparatus.

15. The peripheral device of claim 14, wherein the medical image capturing apparatus comprises a Magnetic Resonance Imaging (MRI) device, and
the peripheral device further comprises a battery charger which is configured to charge the battery by using radio frequency (RF) signals which are generated inside a gantry of the medical image capturing apparatus.
